# EUROPÄISCHE PATENTSCHRIFT

(11) **EP 0 857 466 B1**
(45) Veröffentlichungstag und Bekanntmachung des Hinweises auf die Patenterteilung: **05.06.2002**
(21) Anmeldenummer: 97121020.8
(22) Anmeldetag: 29.11.1997
(51) Int. Cl.: A61B 17/88, A61B 17/58

(54) **Instrument zum Spannen eines Knochenelemente fixierenden Fixierungselementes**
Instrument for tensioning a bone element fixator
Instrument de tension d'un dispositif de fixation d'éléments osseux

(30) Priorität: 09.01.1997 DE 19700474
(43) Veröffentlichungstag der Anmeldung: 12.08.1998
(73) Patentinhaber: Aesculap AG & Co. KG, 78532 Tuttlingen (DE)
(72) Erfinder: Morales, Pedro, D-78532 Tuttlingen (DE); Weisshaupt, Dieter, D-78194 Immendingen (DE)
(74) Vertreter: Hoeger, Stellrecht & Partner

(56) Entgegenhaltungen:
- EP-A- 0 452 623
- WO-A-83/00010
- WO-A-95/05127
- FR-A- 2 717 674
- US-A- 5 057 113
- US-A- 5 370 646

## Beschreibung

Die Erfindung betrifft ein zangenartiges chirurgisches Instrument zum Spannen eines Knochenelemente fixierenden Fixierungselementes mit zwei an einem Grundkörper gelagerten, relativ zueinander verschwenkbaren Branchen und mit einem Arbeitsglied, das zum Spannen des Fixierungselementes durch Verschwenken der Branchen bewegbar ist, wobei der Grundkörper ein Führungselement umfaßt und das Arbeitsglied zwei an das Fixierungselement anlegbare Spannbacken aufweist, die am Führungselement in axialer Richtung des chirurgischen Instrumentes verschieblich gehalten und durch Verschwenken der beiden Branchen mit einer axialen Zugkraft beaufschlagbar sind.

Derartige Instrumente kommen insbesondere bei craniochirurgischen Eingriffen zum Einsatz, bei denen es in vielen Fällen erforderlich ist, der Schädelkapsel eines Patienten ein Kalottensegment zu entnehmen, um auf diese Weise einen Zugang zum darunterliegenden Operationsgebiet zu schaffen. Am Ende der Operation wird das zuvor entnommene Kalottensegment wieder in die verbliebene Schädelkalotte eingesetzt und relativ zu dieser fixiert. Hierzu kommen Fixierungselemente zum Einsatz, bei deren Applikation die voranstehend beschriebenen Instrumente Verwendung finden. Mit diesen können die Fixierungselemente derart verspannt werden, daß das wieder eingesetzte Kalottensegment an der verbliebenen Schädelkalotte fixiert ist.

Üblicherweise kommen zu diesem Zweck chirurgische Instrumente zum Einsatz, die nach Art von Blindnietzangen ausgestaltet sind. Durch Verschwenken der Branchen der Blindnietzangen kann ein Arbeitsglied relativ zu einem Grundkörper bewegt und dadurch das Fixierungselement gespannt werden. Nachteilig an derartigen Instrumenten ist, daß sie dem Operateur bei der Applikation des Fixierungselementes nur eine eingeschränkte Sicht auf das Fixierungselement ermöglichen. Dies ist insbesondere dann der Fall, wenn das Einsetzen des zuvor entnommenen Kalottensegmentes durch bei dessen Entfernung weggespreizte Kopfmuskelschichten und die ebenfalls seitlich abgelegte Kopfhaut behindert wird.

Aus der US-A-5 057 113 ist ein chirurgisches Instrument bekannt, bei dem zwei Branchen an einem Grundkörper gehalten sind, der ein Führungselement aufweist. Am Führungselement sind zwei an das Fixierungselement anlegbare Spannbacken in axialer Richtung des chirurgischen Instrumentes verschiebbar gehalten. Die Branchen sind relativ zueinander verschwenkbar, und durch Verschwenken der Branchen können die Spannbacken mit einer axialen Zugkraft beaufschlagt werden.

Eine derartige Ausgestaltung ermöglicht zwar während der Applikation eine freie Sicht auf das Fixierungselement, es besteht aber die Gefahr, daß bei einer kräftigen Verschwenkbewegung das zu spannende Fixierungselement mit einer unzulässig hohen Zugkraft beaufschlagt wird, die eine Beschädigung des Fixierungselementes zur Folge hat.

Aufgabe der vorliegenden Erfindung ist es, ein zangenartiges chirurgisches Instrument der gattungsgemäßen Art derart auszugestalten, daß bei seiner Handhabung eine Beschädigung des Fixierungselementes vermieden wird.

Diese Aufgabe wird bei einem zangenartigen chirurgischen Instrument der eingangs genannten Art erfindungsgemäß dadurch gelöst, daß das chirurgische Instrument ein die von den beiden Branchen auf die Spannbacken ausübbare Zugkraft begrenzendes Kraftbegrenzungselement umfaßt. Durch das Kraftbegrenzungselement kann sichergestellt werden, daß die beiden Spannbacken und über diese das zu spannende Fixierungselement nicht mit einer unzulässigen Zugkraft beaufschlagt werden, die eine Beschädigung des Fixierungselementes zur Folge hätte.

Bei einer bevorzugten Ausgestaltung ist vorgesehen, daß das Arbeitsglied mit dem Grundkörper lösbar verbindbar ist. So kann beispielsweise vorgesehen sein, daß das Arbeitsglied mit dem Grundkörper verschraubbar ist. Dadurch kann das chirurgische Instrument auf einfache Weise für Reinigungszwecke demontiert werden. Es wird somit bei einfacher Handhabung den hohen hygienischen Anforderungen insbesondere in der Neurochirurgie gerecht.

Bei einer konstruktiv besonders einfachen Ausgestaltung ist vorgesehen, daß das Führungselement als in axialer Richtung ausgerichteter Führungszylinder ausgebildet ist, in welchem die Spannbacken verschieblich gehalten sind. Der Führungszylinder nimmt die Spannbacken auf und dient zu deren Führung. Zur Reinigung und Sterilisation können die Spannbacken dem Führungszylinder entnommen werden.

Bei der Applikation wird das zu spannende Fixierungselement in den Bereich zwischen den beiden einander gegenüberliegenden Spannbacken eingeführt und anschließend von diesen ergriffen. Die Spannbacken können anschließend durch Verschwenken der beiden Branchen in proximale Richtung verschoben werden, so daß das Fixierungselement gespannt wird. Das Einführen des Fixierungselementes zwischen die beiden Spannbacken wird bei einer vorteilhaften Konstruktion dadurch erleichtert, daß das Arbeitsglied ein Halteelement umfaßt, an dem die Spannbacken um eine quer zur Axialrichtung ausgerichtete Achse verschwenkbar gelagert sind. Die Spannbacken können auf diese Weise eine Öffnungs- und Schließbewegung quer zur Axialrichtung des chirurgischen Instrumentes ausführen, wodurch das Ergreifen des Fixierungselementes beträchtlich vereinfacht wird.

Eine weitere Vereinfachung der Handhabung des erfindungsgemäßen chirurgischen Instrumentes kann dadurch erzielt werden, daß die Spannbacken gegen die Wirkung einer elastischen Rückstellkraft um eine quer zur Axialrichtung ausgerichtete Achse verschwenkbar sind. Bei einer derartigen Konstruktion werden die Spannbakken beim Verschwenken von einem beispielsweise als Schraubenfeder ausgebildeten Federelement mit einer in Richtung auf ihre geschlossene Stellung weisenden Federkraft beaufschlagt. Zum Ergreifen eines Fixierungselementes können die Spannbacken gegen die Wirkung des Federelementes in ihre geöffnete Stellung verschwenkt werden.

Wie voranstehend beschrieben, ist es von Vorteil, wenn die beiden Spannbacken an einem Halteelement verschwenkbar gelagert sind. Günstig ist es, wenn das Arbeitsglied ein am Führungselement verschiebbar gehaltenes Schiebeteil umfaßt, das das Halteelement trägt. Das Halteelement stellt somit eine mechanische Verbindung her zwischen den im distalen Endbereich des chirurgischen Instrumentes angeordneten Spannbacken und dem Schiebeteil, das durch Verschwenken der beiden Branchen in axialer Richtung verschiebbar ist.

Um die Reinigung und Sterilisation des erfindungsgemäßen chirurgischen Instrumentes zu vereinfachen, ist es von Vorteil, wenn das Arbeitsglied eine die Spannbacken und das Halteelement umhüllende Abdeckung umfaßt, die mit dem Schiebeteil lösbar verbindbar ist. Dies ermöglicht es, das Arbeitsglied, nachdem es dem Grundkörper entnommen wurde, auf einfache Weise weiter zu zerlegen. Hierzu kann beispielsweise vorgesehen sein, daß die Abdeckung mit dem Schiebeteil verschraubbar ist.

Die Abdeckung hat sich insbesondere in den Fällen als vorteilhaft erwiesen, in denen zur Beaufschlagung der Spannbacken mit einer elastischen Rückstellkraft ein beispielsweise als Schraubenfeder ausgebildetes Federelement zum Einsatz kommt. Dieses kann zwischen dem distalen Ende des Schiebeteiles und einer quer zur Axialrichtung ausgerichteten Anlagefläche des Halteelementes eingespannt sein und das Halteelement mit einer axialen Federkraft beaufschlagen. Diese Federkraft kann durch im Bereich des distalen Endes der Spannbacken vorgesehene Umlenkmittel, die mit den Spannbacken in Wirkverbindung stehen, derart umgelenkt werden, daß die am Halteelement gelagerten Spannbacken beim Verschwenken eine elastische Rückstellkraft erfahren. Die Umlenkmittel können beispielsweise dadurch ausgebildet sein, daß die Abdeckung in ihrem distalen Endbereich innenseitig eine schräg zur Längsachse des Führungselements ausgerichtete Führungsfläche aufweist, an der die Spannbakken anliegen. Durch den schrägen Verlauf der Führungsfläche wird die axiale Federkraft des Federelements in eine quer zur Längsrichtung des Führungselements und damit einer Öffnungsbewegung der Spannbacken entgegengerichtete Rückstellkraft umgelenkt.

Das Federelement kann ebenso wie die Spannbacken und das Halteelement von der Abdeckung umhüllt werden, so daß das gesamte Arbeitselement eine glatte Außenfläche aufweist und auf einfache Weise dem Grundkörper entnommen werden kann.

Von Vorteil ist es, wenn das Arbeitsglied ein verschieblich am Führungselement gehaltenes Verbindungsteil umfaßt, das mit einem an den beiden Branchen verschwenkbar gelagerten Kraftübertragungselement lösbar verbindbar ist. Die von den beiden Branchen auf die Spannbacken ausgeübte Zugkraft wird bei einer derartigen Ausgestaltung über das Kraftübertragungselement und das Verbindungsteil übertragen. Zur Demontage des chirurgischen Instrumentes kann das Verbindungsteil vom Kraftübertragungselement auf einfache Weise gelöst werden. Hierzu kann beispielsweise eine Schraubverbindung zum Einsatz kommen.

Von Vorteil ist es, wenn das Kraftübertragungselement einen das Verbindungsteil in Umfangsrichtung umgebenden Haltering umfaßt sowie zwei Hebelarme, die an einem ersten Anlenkpunkt jeweils an einer Branche und an einem zweiten Anlenkpunkt am Haltering verschwenkbar gelagert sind.

Günstig ist es, wenn der Haltering ein mit einem entsprechenden Außengewinde des Verbindungsteiles korrespondierendes Innengewinde umfaßt. Dies ermöglicht eine Ausgestaltung, bei der das gesamte Arbeitsglied dadurch dem Grundkörper entnommen werden kann, daß das Verbindungsteil aus dem Kraftübertragungselement herausgeschraubt und anschließend das am Verbindungsteil gehaltene Schiebeteil ebenso wie das Halteelement und die Spannbacken aus dem Führungselement herausgezogen werden.

Vorzugsweise umfaßt das Kraftbegrenzungselement ein in den Kraftübertragungsweg zwischen den Branchen und den Spannbacken geschaltetes Federelement, beispielsweise eine Schraubenfeder. Bei einer derartigen Ausgestaltung wird die durch Verschwenken der beiden Branchen ausgeübte Zugkraft nicht ausschließlich über starr ausgebildete Zwischenglieder auf die Spannbacken übertragen, sondern auch über das elastische Federelement, durch das die von den Branchen auf die Spannbacken übertragbare Zugkraft begrenzt wird.

Das Federelement kann vorteilhafterweise als Tellerfedersäule ausgebildet sein. Deren Federkonstante ist durch Wahl der entsprechenden Tellerfedern auf einfache Weise veränderbar. Je nach zum Einsatz kommenden Fixierungselementen kann ein an deren mechanische Belastbarkeit angepaßtes Kraftbegrenzungselement zum Einsatz kommen.

Bei einer kostengünstig herstellbaren Ausführungsform ist vorgesehen, daß sich das Federelement einerseits am Verbindungsteil und andererseits an einer verschieblich am Verbindungsteil gehaltenen Zugstange abstützt, die an dem sich in distaler Richtung an das Verbindungsteil anschließenden Schiebeteil festgelegt ist. Beim Verschwenken der beiden Branchen werden das Verbindungsteil und das sich an diesem abstützende Federelement in proximale Richtung verschoben. Dies hat zur Folge, daß vom Federelement eine in proximale Richtung gerichtete Zugkraft auf die Zugstange übertragen wird, die wiederum das Schiebeteil und die mit diesem lösbar verbundene Abdeckung mit einer entsprechenden Zugkraft beaufschlagt. Von der Abdeckung kann die Zugkraft über die schräg zur Längsachse ausgerichtete Führungsfläche auf die Spannbacken übertragen werden. Beim Betätigen des erfindungsgemäßen chirurgischen Instrumentes legen allerdings das Verbindungsteil und das über die Zugstange mit diesem verbundene Schiebeteil nur so lange den gleichen Arbeitsweg zurück, bis ein Maximalwert der Zugkraft erzielt wird. Eine weitere Erhöhung der Zugkraft hat lediglich zur Folge, daß das Federelement zunehmend belastet wird, eine weitere Verschiebung des Schiebeteils und damit auch der Spannbacken in proximale Richtung jedoch praktisch nicht erfolgt.

Eine weitere Begrenzung der von den beiden Branchen ausübbaren Zugkraft wird bei einer vorteilhaften Ausgestaltung dadurch erzielt, daß das chirurgische Instrument einen den Verschwenkbereich der beiden Branchen begrenzenden Anschlag umfaßt. Dieser kann beispielsweise an den dem Arbeitsglied zugewandten Innenseiten der beiden Branchen angeordnet sein.

Um die Handhabung des erfindungsgemäßen chirurgischen Instrumentes zusätzlich zu erleichtern, ist bei einer bevorzugten Ausgestaltung vorgesehen, daß die beiden Branchen gegen die Wirkung einer elastischen Rückstellkraft verschwenkbar sind. So kann beispielsweise vorgesehen sein, daß die dem Grundkörper abgewandten freien Enden der beiden Branchen über eine Blattfeder miteinander verbunden sind.

Die nachfolgende Beschreibung einer bevorzugten Ausführungsform dient im Zusammenhang mit der Zeichnung der näheren Erläuterung. Es zeigen:
- Figur 1:: eine schematische, teilweise aufgebrochen dargestellte Draufsicht auf ein erfindungsgemäßes chirurgisches Instrument;
- Figur 2:: eine vergrößerte Darstellung des distalen Endbereiches des chirurgischen Instrumentes bei der Applikation eines Fixierungselementes;
- Figur 3:: eine vergrößerte Darstellung entsprechend Figur 2 beim Verschwenken der beiden Branchen des chirurgischen Instrumentes und
- Figur 4:: eine vergrößerte Darstellung entsprechend Figur 2 beim Erreichen einer Endstellung der beiden Branchen.

In der Zeichnung ist ein insgesamt mit dem Bezugszeichen 10 belegtes zangenartiges chirurgisches Instrument dargestellt mit zwei Branchen 12, 14, die an ihrem distalen Ende jeweils an einer seitlich von einem axial ausgerichteten Führungszylinder 16 abstehenden Halterippe 18 bzw. 20 angelenkt und jeweils um eine quer zur Längsachse des Führungszylinders 16 ausgerichtete Achse verschwenkbar sind. An ihrem freien, dem Führungszylinder 16 abgewandten proximalen Ende sind die beiden Branchen 12 und 14 über eine Blattfeder 22 miteinander verbunden, die zwei Schenkel 24 und 26 aufweist, die im distalen Endbereich der Branchen 12, 14 an deren einander gegenüberliegenden Innenseiten 28 bzw. 30 mittels Schrauben 32 bzw. 34 verschraubt sind. Durch die Blattfeder 22 werden die beiden Branchen 12 und 14 des chirurgischen Instrumentes 10 beim Verschwenken mit einer elastischen Rückstellkraft beaufschlagt.

Der Führungszylinder 16 verjüngt sich in seinem distalen Endbereich, während er über seine restliche Längserstreckung im wesentlichen kreiszylinderförmig ausgestaltet ist. In seinem distalen Endbereich trägt der Führungszylinder 16 ein Innengewinde 36, in das ein im wesentlichen pilzförmiges Anlageelement 38 mit einer zentralen Durchgangsbohrung 40 eingeschraubt ist.

In proximaler Richtung schließt sich an das Innengewinde 36 des Führungszylinders 16 eine kreiszylinderförmige Aufnahme 42 an, deren Durchmesser größer ist als der Durchmesser des Innengewindes 36 und die in eine dem Anlageelement 38 abgewandte und quer zur Längsachse des Führungszylinders 16 ausgerichtete Endfläche 44 einmündet.

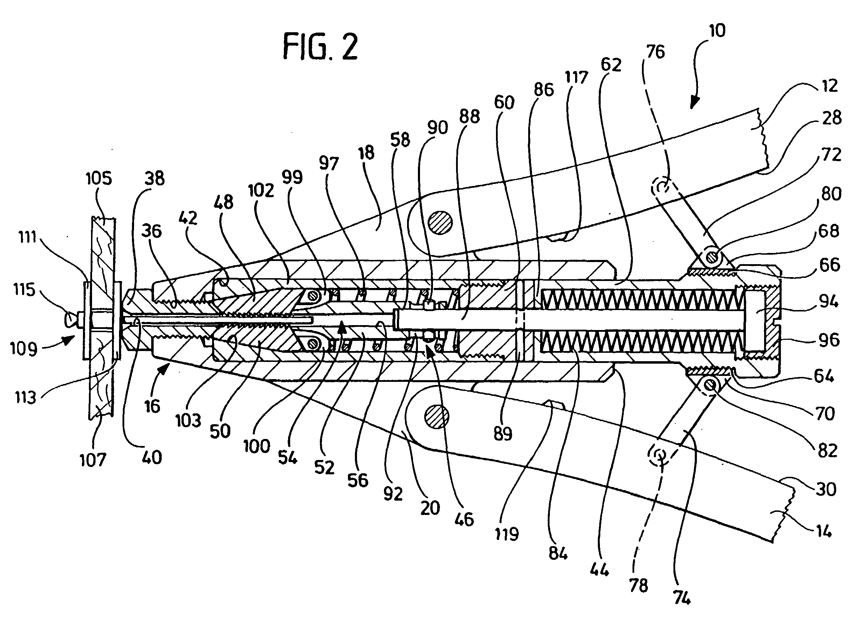

Die Aufnahme 42 des Führungszylinders 16 nimmt ein insgesamt mit dem Bezugszeichen 46 belegtes Arbeitsglied auf, das in der Aufnahme 42 verschieblich gehalten ist. Das Arbeitsglied 46 umfaßt an seinem distalen, dem Anlageelement 38 benachbarten Ende zwei keilförmige, sich in distale Richtung verjüngende Spannbacken 48 und 50, die jeweils um eine quer zur Längsachse des Führungszylinders 16 ausgerichtete Achse drehbar an einem im wesentlichen zylinderförmigen Halteelement 52 gelagert sind. Das Halteelement 52 weist eine mit der Durchgangsbohrung 40 des Anlageelementes 38 fluchtende Axialbohrung 54 auf, die sich in proximaler Richtung stufig erweitert und einen distalen Bereich 56 umfaßt, dessen Innendurchmesser im wesentlichen dem Innendurchmesser der Durchgangsbohrung 40 des Anlageelementes 38 entspricht, und einen proximalen Bereich 58 mit größerem Durchmesser (siehe Figuren 2, 3 und 4).

In proximaler Richtung schließt sich an das Halteelement 52 ein Schiebeteil in Form eines zylinderförmigen Kolbens 60 an, dessen Außendurchmesser dem Innendurchmesser der Aufnahme 42 entspricht und der in der Aufnahme 42 verschieblich gehalten ist. An den Kolben 60 wiederum schließt sich in proximaler Richtung eine im wesentlichen kreiszylinderförmige Hülse 62 an, die in ihrem proximalen Endbereich ein Außengewinde 64 trägt und über dieses mit einem Haltering 66 verschraubt ist, von dem zwei Lagerrippen 68 und 70 einander diagonal gegenüberliegend abstehen. Die Lagerrippen 68 und 70 sind über Hebelarme 72 bzw. 74 mit den Branchen 12 bzw. 14 gelenkig verbunden. Zu diesem Zweck sind die Hebelarme 72 und 74 endseitig jeweils an einer ersten Lagerstelle 76 bzw. 78 an den Branchen 12 bzw. 14 und an einer zweiten Lagerstelle 80 bzw. 82 an den Lagerrippen 68 bzw. 70 verschwenkbar gelagert.

Die Hülse 62 umgibt eine Tellerfedersäule 84, die sich distalseitig an einer Bodenwand 86 der Hülse 62 abstützt und ihrerseits eine Zugstange 88 umgibt, die die Tellerfedersäule 84 ebenso wie den Kolben 60 durchgreift und in den proximalen Bereich 58 der Axialbohrung 54 des Halteelementes 52 eintaucht. Die Zugstange 88 wird in ihrem distalen Endbereich von einem abstehenden Sicherungsstift 90 durchgriffen, der in einer quer zur Axialbohrung 54 ausgerichteten Führungsbohrung 92 des Halteelementes 52 geführt ist. Mittels eines den Kolben 60 und die Zugstange 88 durchgreifenden Sicherungsbolzens 89 ist die Zugstange 88 unverschieblich am Kolben 60 festgelegt.

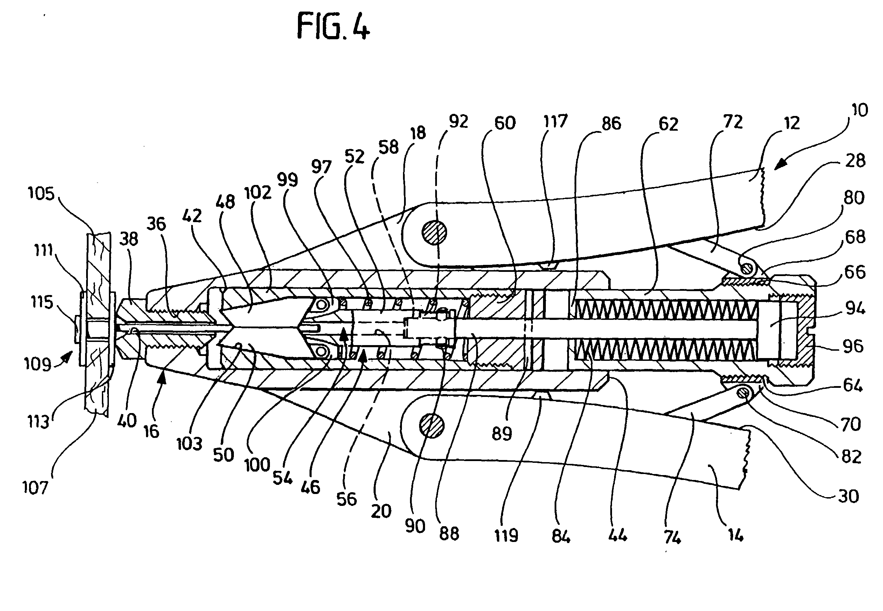

An ihrem proximalen Ende trägt die Zugstange 88 einen an der Tellerfedersäule 84 anliegenden Zugkopf 94, dessen Außendurchmesser geringfügig geringer gewählt ist als der Innendurchmesser der Hülse 62, so daß der Zugkopf 94 an der Innenseite der Hülse 62 englanggleiten kann. Dies wird insbesondere aus Figur 4 deutlich.

Auf ihrer proximalen Stirnseite wird die Hülse 62 durch eine in diese eingeschraubte Madenschraube 96 verschlossen, die den Zugkopf 94 in Umfangsrichtung umgibt.

Der in die Axialbohrung 54 eintauchende distale Endbereich der Zugstange 88 und das Halteelement 52 sind von einer Schraubenfeder 97 umgeben, die sich einerseits an einer distalen Stirnfläche des Kolbens 60 und andererseits an seitlich vorstehenden Querrippen 99 und 100 des Halteelementes 52 abstützt, an denen die Spannbakken 48 bzw. 50 angelenkt sind. Durch die Schraubenfeder 97 werden die Spannbacken 48 und 50 mit einer in distale Richtung weisenden Federkraft beaufschlagt.

Die Spannbacken 48 und 50 werden ebenso wie die Schraubenfeder 97 von einer auf den Kolben 60 aufgeschraubten Abdeckhülse 102 umhüllt. Die Abdeckhülse 102 bildet in ihrem distalen Endbereich innenseitig eine schräg zur Längsachse des Führungszylinders 16 ausgerichtete Führungsfläche 103, indem sich die Durchgangsbohrung der Abdeckhülse 102 in diesem Bereich konisch verjüngt. Die im wesentlichen keilförmig ausgestalteten Spannbacken 48 und 50 gleiten aufgrund der in distale Richtung weisenden Federkraft der Schraubenfeder 97 an der Führungsfläche 103 der Abdeckhülse 102 entlang und werden dadurch beim Verschwenken mit einer elastischen Rückstellkraft beaufschlagt.

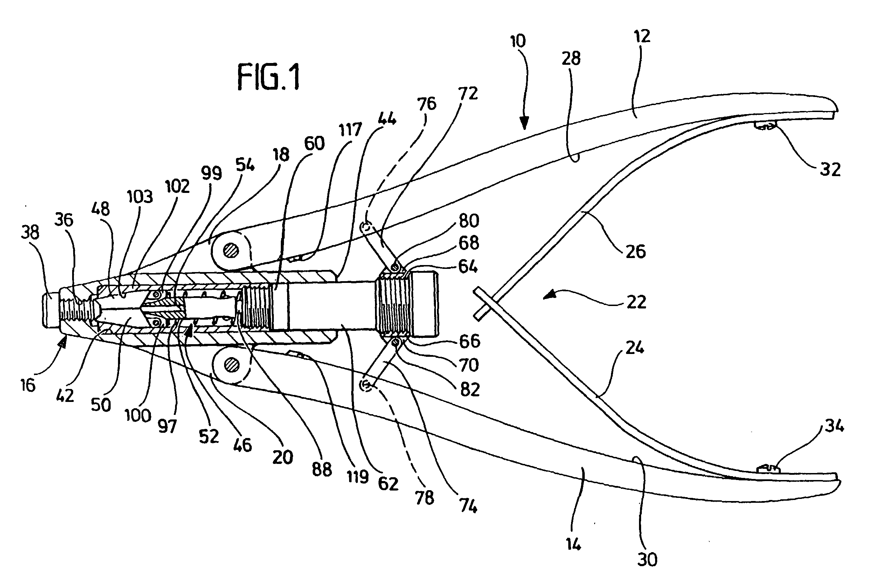

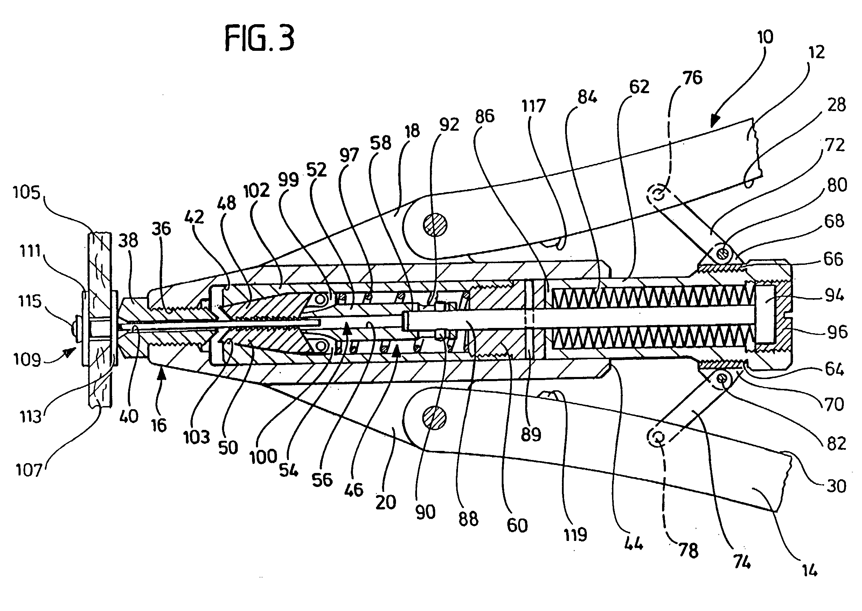

Das erfindungsgemäße chirurgische Instrument 10 kann insbesondere zur Fixierung zweier Knochenelemente mittels eines Fixierungselementes verwendet werden. Dies ist schematisch in den Figuren 2 bis 4 dargestellt, in denen zwei Knochenelemente mit den Bezugszeichen 105 und 107 belegt sind. Die Knochenelemente 105 und 107 können mittels eines Fixierungselementes 109 gegenseitig festgelegt werden. Das Fixierungselement 109 umfaßt zwei Fixierplatten 111 und 113 sowie einen die beiden Fixierplatten 111 und 113 durchgreifenden Niet 115. Durch Spannen des Nietes 115 können die beiden Fixierplatten 111 und 113 an den Knochenelementen 105 und 107 festgelegt werden und dadurch die beiden Knochenelemente 105 und 107 in ihrer gegenseitigen Lage fixiert werden. Zum Spannen des Fixierungselementes 109 kann der Niet 115 durch die Durchgangsbohrung 40 des Anlageelementes 38 hindurch in den Bereich zwischen den beiden Spannbacken 48 und 50 eingeführt werden. Da die Spannbacken 48 und 50 an der schräg ausgerichteten Führungsfläche 103 entlanggleiten, wird die in distale Richtung weisende Federkraft der Schraubenfeder 97 derart umgelenkt, daß der Niet 115 von den beiden Spannbacken 48 und 50 ergriffen wird. Anschließend können die beiden Branchen 12 und 14 aus ihrer in den Figuren 1 und 2 dargestellten geöffneten Stellung über die in Figur 3 abgebildete Zwischenposition in die in Figur 4 dargestellte geschlossene Stellung verschwenkt werden. Das Verschwenken der beiden Branchen 12 und 14 hat zur Folge, daß die Hülse 62 über die Hebelarme 72 und 74 in proximale Richtung verschoben wird. Die somit erzeugte Zugkraft wird über die Tellerfedersäule 84 auf den Zugkopf 94 der Zugstange 88 und von dieser auf den Kolben 60 und die Abdeckhülse 102 und über die Führungsfläche 103 schließlich auf die Spannbacken 48 und 50 übertragen. Dadurch werden die Spannbacken 48 und 50 - wie in Figur 3 dargestellt - in proximale Richtung verschoben und damit der Niet 115 gespannt. Da die Hülse 62 nicht starr mit dem Kolben 60 verbunden ist, sondern die Zugkraft über die Tellerfedersäule 84 übertragen wird, kann von den beiden Branchen 12 und 14 nur eine begrenzte Zugkraft auf den Kolben 60 und über diesen auf die Spannbacken 48 und 50 übertragen werden, da bei zunehmender Kraftbeaufschlagung die Tellerfedersäule 84 zusammengedrückt wird, ohne daß dabei der Kolben 60 und die Spannbacken 48 und 50 in proximale Richtung um den gleichen Arbeitsweg verschoben werden wie die Hülse 62. In den Figuren 3 und 4 wird dies dadurch deutlich, daß sich die Hülse 62 von einer proximalen Endfläche des Kolbens 60 ablöst. Die Tellerfedersäule 84 wirkt somit als Kraftbegrenzungselement.

Die Verschwenkbewegung der beiden Branchen 12 und 14 wird schließlich durch an den Innenseiten 28 bzw. 30 in Höhe des Führungszylinders 16 angeordnete Anschläge 117 und 119 begrenzt, die in der geschlossenen Stellung der beiden Branchen 12 und 14 an der Außenseite des Führungszylinders 16 zur Anlage kommen.

Zur Reinigung und Sterilisation des chirurgischen Instrumentes 10 kann das Arbeitsglied 46 dem Führungszylinder 16 entnommen werden. Hierzu ist es lediglich erforderlich, die Schraubverbindung zwischen der Hülse 62 und dem Haltering 66 zu lösen und anschließend das Arbeitsglied 46 aus der Aufnahme 42 herauszuziehen.

Das Arbeitsglied 46 kann dann zur Reinigung und Sterilisation weiter zerlegt werden, indem die Abdeckhülse 102 vom Kolben 60 abgeschraubt wird, so daß sich ein unmittelbarer Zugang ergibt auf die Schraubenfeder 97 und die Querrippen 99 und 100, an denen die Spannbacken 48 und 50 verschwenkbar gehalten sind. Um beim Abschrauben der Abdeckhülse 102 vom Kolben 60 zu vermeiden, daß sich das Halteelement 52 aufgrund der Federkraft der Schraubenfeder 97 vollständig vom Kolben 60 löst, ist das Halteelement über den Sicherungsstift 90 und das distale Ende der Zugstange 88 am Kolben 60 gehalten.

Das chirurgische Instrument 10 zeichnet sich insbesondere durch eine axiale Arbeitsrichtung aus. Dies hat den Vorteil, daß dem Operateur beim Einsatz des chirurgischen Instrumentes 10 die Sicht auf das Fixierungselement 109 nicht behindert wird.

## Patentansprüche

1. Zangenartiges chirurgisches Instrument zum Spannen eines Knochenelemente fixierenden Fixierungselementes mit zwei an einem Grundkörper gelagerten, relativ zueinander verschwenkbaren Branchen (12, 14) und mit einem Arbeitsglied (46), das zum Spannen des Fixierungselementes (109) durch Verschwenken der Branchen (12, 14) bewegbar ist, wobei der Grundkörper ein Führungselement (16) umfaßt und das Arbeitsglied (46) zwei an das Fixierungselement (109) anlegbare Spannbacken (48, 50) aufweist, die am Führungselement (16) in axialer Richtung des chirurgischen Instrumentes (10) verschieblich gehalten sind und durch Verschwenken der beiden Branchen (12, 14) mit einer axialen Zugkraft beaufschlagbar sind, **dadurch gekennzeichnet, daß** das chirurgische Instrument (10) ein die von den beiden Branchen (12, 14) auf die Spannbacken (48, 50) ausübbare Zugkraft begrenzendes Kraftbegrenzungselement (84) umfaßt.

2. Chirurgisches Instrument nach Anspruch 1, **dadurch gekennzeichnet, daß** das Arbeitsglied (46) mit dem Grundkörper lösbar verbindbar ist.

3. Chirurgisches Instrument nach Anspruch 1 oder 2, **dadurch gekennzeichnet, daß** das Führungselement als in axialer Richtung ausgerichteter Führungszylinder (16) ausgebildet ist, in dem die Spannbacken (48, 50) verschieblich gehalten sind.

4. Chirurgisches Instrument nach Anspruch 1, 2 oder 3, **dadurch gekennzeichnet, daß** das Arbeitsglied (46) ein Halteelement (52) umfaßt, an dem die Spannbacken (48, 50) um eine quer zur Axialrichtung ausgerichtete Achse verschwenkbar gelagert sind.

5. Chirurgisches Instrument nach einem der voranstehenden Ansprüche, **dadurch gekennzeichnet, daß** die Spannbacken (48, 50) gegen die Wirkung einer elastischen Rückstellkraft um eine quer zur Axialrichtung ausgerichtete Achse verschwenkbar sind.

6. Chirurgisches Instrument nach Anspruch 4 oder 5, **dadurch gekennzeichnet, daß** das Arbeitsglied (46) ein am Führungselement (16) verschieblich gehaltenes Schiebeteil (60) umfaßt, das das Halteelement (52) trägt.

7. Chirurgisches Instrument nach Anspruch 6, **dadurch gekennzeichnet, daß** das Arbeitsglied (46) eine die Spannbacken (48, 50) und das Halteelement (52) umhüllende Abdeckung (102) umfaßt, die mit dem Schiebeteil (60) lösbar verbindbar ist.

8. Chirurgisches Instrument nach einem der voranstehenden Ansprüche, **dadurch gekennzeichnet, daß** das Arbeitsglied (46) ein verschieblich am Führungselement (16) gehaltenes Verbindungsteil (62) umfaßt, das mit einem an den beiden Branchen (12, 14) verschwenkbar gelagerten Kraftübertragungselement lösbar verbindbar ist.

9. Chirurgisches Instrument nach Anspruch 8, **dadurch gekennzeichnet, daß** das Kraftübertragungselement einen das Verbindungsteil (62) in Umfangsrichtung umgebenden Haltering (66) umfaßt sowie zwei Hebelarme (72, 74), die an einem ersten Anlenkpunkt (76, 78) an jeweils einer Branche (12, 14) und an einem zweiten Anlenkpunkt (80, 82) am Haltering (66) verschwenkbar gelagert sind.

10. Chirurgisches Instrument nach einem der voranstehenden Ansprüche, **dadurch gekennzeichnet, daß** das Kraftbegrenzungselement ein in den Kraftübertragungsweg zwischen den Branchen (12, 14) und den Spannbacken (48, 50) geschaltetes Federelement (84) umfaßt.

11. Chirurgisches Instrument nach Anspruch 10, **dadurch gekennzeichnet, daß** das Federelement als Tellerfedersäule (84) ausgebildet ist.

12. Chirurgisches Instrument nach Anspruch 10 oder 11, **dadurch gekennzeichnet, daß** sich das Federelement (84) einerseits am Verbindungsteil (62) und andererseits an einer verschieblich am Verbindungsteil (62) gehaltenen Zugstange (84) abstützt, die an dem sich in distaler Richtung an das Verbindungsteil (62) anschließenden Schiebeteil (60) festgelegt ist.

13. Chirurgisches Instrument nach einem der voranstehenden Ansprüche, **dadurch gekennzeichnet, daß** das chirurgische Instrument (10) einen den Verschwenkbereich zwischen den beiden Branchen (12, 14) begrenzenden Anschlag (117, 119) umfaßt.

14. Chirurgisches Instrument nach einem der voranstehenden Ansprüche, **dadurch gekennzeichnet, daß** die beiden Branchen (12, 14) gegen die Wirkung einer elastischen Rückstellkraft verschwenkbar sind.

## Claims

1. A forceps-type surgical instrument for tensioning a fixator fixing elements of bone, comprising two handles (12, 14) mounted on a base body and pivotable relative to one another, and an operating element (46) which is movable in order to tension the fixator (109) by the pivoting of the handles (12, 14), the base body comprising a guide element (16), and the operating element (46) having two tensioning jaws (48, 50) which may be applied to the fixator (109) and which are held on the guide element (16) so as to be displaceable in the axial direction of the surgical instrument (10) and may be acted upon with an axial tensile force by the pivoting of the two handles (12, 14), **characterised in that** the surgical instrument (10) comprises a force-limiting element (84) limiting the tensile force which may be exerted by the two handles (12, 14) on the tensioning jaws (48, 50).

2. A surgical instrument according to claim 1, **characterised in that** the operating element (46) may be detachably connected to the base body.

3. A surgical instrument according to claim 1 or 2, **characterised in that** the guide element is a guide cylinder (16), oriented in the axial direction, in which the tensioning jaws (48, 50) are held so as to be displaceable.

4. A surgical instrument according to claim 1, 2 or 3, **characterised in that** the operating element (46) comprises a holding element (52) on which the tensioning jaws (48, 50) are mounted so as to pivot about an axis oriented transversely to the axial direction.

5. A surgical instrument according to one of the preceding claims, **characterised in that** the tensioning jaws (48, 50) are pivotable against the effect of a resilient restoring force about an axis oriented transversely to the axial direction.

6. A surgical instrument according to claim 4 or 5, **characterised in that** the operating element (46) comprises a sliding part (60), held so as to be displaceable on the guide element (16), which sliding part carries the holding element (52).

7. A surgical instrument according to claim 6, **characterised in that** the operating element (46) comprises a covering (102), encasing the tensioning jaws (48, 50) and the holding element (52), which covering may be detachably connected to the sliding part (60).

8. A surgical instrument according to one of the preceding claims, **characterised in that** the operating element (46) comprises a connecting part (62), held so as to be displaceable on the guide element (16), which part may be detachably connected to a force-transfer element pivotally mounted on the two handles (12, 14).

9. A surgical instrument according to claim 8, **characterised in that** the force-transfer element comprises a supporting ring (66), encircling the connecting part (62) in the circumferential direction, and also two lever arms (72, 74) which are pivotally mounted at a first articulation point (76, 78) on the respective handles (12, 14) and at a second articulation point (80, 82) on the supporting ring (66).

10. A surgical instrument according to one of the preceding claims, **characterised in that** the force-limiting element comprises a spring element (84) connected into the force-transfer path between the handles (12, 14) and the tensioning jaws (48, 50).

11. A surgical instrument according to claim 10, **characterised in that** the spring element is a disc spring column (84).

12. A surgical instrument according to claim 10 or 11, **characterised in that** the spring element (84) is supported on the connecting part (62), on the one hand, and on a drawbar (88), on the other hand, which is displaceably held on the connecting part (62) and is secured to the sliding part (60) following the connecting part (62) in the distal direction.

13. A surgical instrument according to one of the preceding claims, **characterised in that** the surgical instrument (10) comprises a stop (117, 119) limiting the pivoting range between the two handles (12, 14).

14. A surgical instrument according to one of the preceding claims, **characterised in that** the two handles (12, 14) are pivotable against the effect of a resilient restoring force.

## Revendications

1. Instrument chirurgical de type pince destiné à serrer un élément de fixation de pièces osseuses, qui comporte deux branches (12, 14), aptes à pivoter l'une par rapport à l'autre et montées sur un corps de basé, et un organe de travail (46) qui est apte à se déplacer en faisant pivoter les branches (12, 14) en vue de serrer l'élément de fixation (109) ; le corps de base comportant un élément de guidage (16), et l'organe de travail (46) comportant deux mâchoires de serrage (48, 50), aptes à être montées sur l'élément de fixation (109), qui sont maintenues sur l'élément de guidage (16) de façon à être déplaçables axialement par rapport à l'instrument chirurgical (10) et qui peuvent être soumises à une force de traction axiale en faisant pivoter les deux branches (12, 14), **caractérisé en ce que** l'instrument chirurgical (10) comporte un élément de limitation de force (84) dont le but est de limiter la force de traction qui peut être exercée par les deux branches (12, 14) sur les mâchoires de serrage (48, 50).

2. Instrument chirurgical selon la revendication 1, **caractérisé en ce que** l'organe de travail (46) peut être relié de façon amovible au corps de base.

3. Instrument chirurgical selon la revendication 1 ou 2, **caractérisé en ce que** l'élément de guidage est conformé en cylindre de guidage (16), orienté axialement, dans lequel les mâchoires de serrage (48, 50) sont maintenues de façon à être déplaçables.

4. Instrument chirurgical selon la revendication 1, 2 ou 3, **caractérisé en ce que** l'organe de travail (46) comporte un élément de maintien (52) sur lequel les mâchoires de serrage (48, 50) sont montées de façon à pouvoir pivoter autour d'un axe orienté transversalement à la direction axiale.

5. Instrument chirurgical selon l'une des revendications précédentes, **caractérisé en ce que** les mâchoires de serrage (48, 50) sont aptes à pivoter autour d'un axe orienté transversalement à la direction axiale, en s'opposant à l'action d'une force de rappel élastique.

6. Instrument chirurgical selon la revendication 4 ou 5, **caractérisé en ce que** l'organe de travail (46) comporte une pièce coulissante (60), maintenue de façon déplaçable sur l'élément de guidage (16), qui supporte l'élément de maintien (52).

7. Instrument chirurgical selon la revendication 6, **caractérisé en ce que** l'organe de travail (46) comporte un élément de recouvrement (102), entourant les mâchoires de serrage (48, 50) et l'élément de maintien (52), qui peut être relié de façon amovible à la pièce coulissante (60).

8. Instrument chirurgical selon l'une des revendications précédentes, **caractérisé en ce que** l'organe de travail (46) comporte une pièce de liaison (62), maintenue de façon déplaçable sur l'élément de guidage (16), qui peut être reliée de façon amovible à un élément de transfert de force monté sur les deux branches (12, 14) de façon à pouvoir pivoter.

9. Instrument chirurgical selon la revendication 8, **caractérisé en ce que** l'élément de transfert de force comporte une bague de blocage (66) entourant périphériquement la pièce de liaison (62), ainsi que deux bras de levier (72, 74) qui sont montés de façon à pouvoir pivoter autour d'un premier point d'articulation (76, 78) sur chacune des branches (12, 14) et d'un second point d'articulation (80, 82) sur la bague de blocage (66).

10. Instrument chirurgical selon l'une des revendications précédentes, **caractérisé en ce que** l'élément de limitation de force comporte un élément formant ressort (84) monté dans l'espace de transfert de force entre les branches (12, 14) et les mâchoires de serrage (48, 50).

11. Instrument chirurgical selon la revendication 10, **caractérisé en ce que** l'élément formant ressort est agencé sous forme d'un empilement de rondelles Belleville (84).

12. Instrument chirurgical selon la revendication 10 ou 11, **caractérisé en ce que** l'élément formant ressort (84) est en appui d'un côté sur la pièce de liaison (62) et d'un autre côté sur une tige de traction (88), maintenue de façon déplaçable sur la pièce de liaison, qui est fixée à la pièce coulissante (60) se raccordant dans la direction distale à la pièce de liaison (62).

13. Instrument chirurgical selon l'une des revendications précédentes, **caractérisé en ce que** l'instrument chirurgical (10) comporte une butée (117, 119) limitant la plage de pivotement entre les deux branches (12, 14).

14. Instrument chirurgical selon l'une des revendications précédentes, **caractérisé en ce que** les deux branches (12, 14) sont aptes à pivoter en s'opposant à une force de rappel élastique.
